Europäisches Patentamt

European Patent Office

Office européen des brevets

(19)

(11) Numéro de publication: **0 124 393**
**A1**

# (12) DEMANDE DE BREVET EUROPEEN

(21) Numéro de dépôt: **84400609.8**

(22) Date de dépôt: **27.03.84**

(51) Int. Cl.³: **A 61 K 7/13**

(30) Priorité: **01.04.83 FR 8305414**

(43) Date de publication de la demande: **07.11.84**
**Bulletin 84/45**

(84) Etats contractants désignés: **AT BE CH DE FR GB IT LI LU NL SE**

(71) Demandeur: **ALBAN MULLER INTERNATIONAL, S.A.R.L., 19 rue Saint Just, F-93100 Montreuil (FR)**

(72) Inventeur: **Melin, Christian, 361 rue aux Chiens, F-45160 Olivet (FR)**

(74) Mandataire: **Lemoine, Michel et al, Cabinet Michel Lemoine 13 Boulevard des Batignolles, F-75008 Paris (FR)**

(54) Composition et procédé pour la teinture du système pileux.

(57) Composition et procédé pour la teinture du système pileux.

Nouvelle composition de teinture des cheveux et du système pileux, contenant en mélange des colorants naturels liquides d'origine végétale complexés par des cations métalliques et un support d'origine naturelle.

EP 0 124 393 A1

La présente invention a pour objet une composition pour la teinture semi-permanente et réversible des cheveux et du système pileux, faisant appel uniquement à des colorants naturels d'origine végétale.

Plus particulièrement, la présente invention se propose de fournir une composition se présentant sous la forme d'extraits végétaux colorants liquides et d'un support conduisant, après mélange des deux, à une préparation de teinture, aisée à appliquer sur l'ensemble de la chevelure ou sur une zone précise du système pileux.

Depuis la découverte des colorants synthétiques, la teinture des cheveux et du système pileux est devenue courante et est réalisée à l'aide de préparations aqueuses de colorants utilisées directement ou après dilution à l'aide d'agents révélateurs, généralement des agents oxydants puissants. Ces préparations se présentent le plus souvent sous forme de crèmes, shampooings, gels, de consistance plus ou moins ferme.

Les molécules colorantes utilisées sont actuellement pour la plupart d'origine synthétique permettant d'obtenir, en mélanges, une grande variété de nuances. Ces derniers sont soit des colorants "d'oxydation", c'est-à-dire dont le pouvoir colorant ne se développe qu'après action d'un agent d'oxydation, soit des colorants directs teintant le cheveu sans addition d'agent révélateur.

Il existe, en plus de cette catégorie, des colorants dits naturels, d'origine végétale ou animale. Ces colorants sous forme d'extraits de plantes, ou sous forme de molécules isolées responsables du pouvoir colorant, présentent l'avantage par rapport aux colorants synthétiques d'être presque toujours inoffensifs et de ne pas entraîner les réactions cutanées désagréables des colorants de synthèse. Ils sont utilisés à l'heure actuelle pour la coloration du cheveu mais le plus souvent associés à des colorants synthétiques qui confèrent à la préparation sa réelle efficacité.

Cet état de fait est la conséquence d'une mauvaise fixation en général du colorant végétal sur le cheveu ou de l'absence dans le milieu d'adjuvants nécessaires à cette fixation ou même d'une trop grande variabilité de la teneur en matières colorantes de l'extrait végétal.

Parmi les extraits colorants d'origine végétale utilisés depuis de très nombreuses années pour la coloration du cheveu, nous pouvons citer ceux de henné (Lawsonia inermis), dont la matière colorante est la 2-hydroxy 1, 4-naphtoquinone (ou lawsone), présente dans les feuilles sous forme de glycoside.

Comme la plupart des colorants d'origine végétale, la lawsone présente l'inconvénient d'être particulièrement instable à la lumière en milieu aqueux, ceci expliquant l'utilisation populaire de la poudre de feuilles de henné directement en cataplasmes sur la chevelure.

En outre, le henné, largement utilisé pour la teinture des cheveux et de la peau dans de nombreux pays, est un des rares colorants d'origine végétale réellement efficaces en coloration directe du cheveu ou du poil, la plupart des autres colorants naturels ne produisant que de simples reflets ou même n'étant pas utilisés à cette fin.

De plus l'utilisation de poudre de plantes, ou d'extraits en poudre de plantes, s'avère difficile étant donné leur faible solubilité dans l'eau.

L'objectif de l'invention est de pallier les inconvénients d'utilisation des colorants naturels d'origine végétale existant actuellement comme teinture capillaire, d'élargir la gamme d'extraits végétaux pouvant teindre le cheveu ou le poil et surtout d'augmenter considérablement le pouvoir tinctorial des colorants naturels d'origine végétale en conservant leur très bonne innocuité.

Ce résultat est obtenu selon l'invention en complexant des extraits colorants naturels liquides d'origine végétale rigoureusement dosés en leurs molécules colorantes, ou des colorants naturels d'origine végétale, par des cations métalliques.

Ces complexes formés ont une affinité beaucoup plus forte pour le cheveu ou le poil, permettant ainsi d'obtenir une teinture utilisable à froid, à fort pouvoir couvrant, reproductible, applicable à de très nombreux colorants naturels et n'abîmant pas le cheveu ou le poil, les cations métalliques étant les intermédiaires nécessaires à l'accrochage des molécules colorantes sur le cheveu ou le poil.

En outre, la présente invention associe les extraits végétaux colorants à des agents auxiliaires de pénétration ainsi qu'à un support apte à favoriser leur application et le contact avec le cheveu ou le poil.

Par colorant naturel d'origine végétale, on doit entendre les colorants d'origine végétale obtenus par extraction ou ces mêmes colorants obtenus par voie synthétique.

Par extrait(s) colorant(s) liquide(s) d'origine végétale, on doit entendre un (des) extrait(s) de plante(s) ou de partie(s) de plante(s) obtenu(s) par extraction à l'aide de solvants rigoureusement choisis dans le but d'obtenir une concentration en matières colorantes la plus élevée possible, et ce au moyen de toutes méthodes d'extraction, de purification et de concentration utiles à cette fin.

Parmi les différents solvants d'extraction utilisés, on peut citer l'eau, les alcools, glycols et leurs dérivés de bas poids moléculaire : alcools du type méthylique, butylique, propylique, isopropylique ; glycols du type propylène glycol, dipropylène glycol, butylène glycol, carbitols, cellosolves, etc... ($<C_{10}$) ainsi que les mélanges des deux ou des trois types de solvants.

Parmi les méthodes d'extraction utilisées, on peut citer la macération, l'infusion, la lixiviation, la décoction, la turbo-extraction, à froid ou à chaud, l'extraction au $CO_2$ liquide.

Parmi les méthodes de concentration et de purification utilisées, on peut citer la centrifugation, la distillation, la chromatographie, la dialyse.

Les extraits colorants liquides d'origine végétale obtenus sont standardisés à une même concentration de matières colorantes complexées par un sel métallique, permettant ainsi d'obtenir la même couleur de cheveu ou de poil avec les mêmes conditions de teinture.

La présente invention a pour objet des compositions cosmétiques sous forme d'extraits végétaux colorants naturels liquides, destinés à la teinture directe semi-permanente et réversible des cheveux et du système pileux, caractérisées par le fait qu'elles sont constituées de :

a) au moins un extrait colorant ou des colorants naturels d'origine végétale dont les molécules tinctoriales sont sous forme de complexes avec un ou plusieurs cations métalliques rigoureusement dosés, associés à un ou plusieurs agents de pénétration en milieu liquide ;

b) au moins un support d'origine naturelle permettant une application efficace du complexe colorant.

Les cations métalliques sont eux aussi rigoureusement dosés afin qu'ils ne soient pas présents en quantité appréciable à l'état libre et en excès dans la préparation.

Les extrait(s) colorant(s) liquide(s) d'origine végétale sont avantageusement présents dans les compositions selon l'invention à une teneur comprise entre 0,01 et 50 % et de préférence entre 1 et 20 %, en poids par rapport au poids total de la composition.

Ils peuvent être obtenus par extraction de tiges, d'écorces, de fleurs, de racines, de fruits, de feuilles, de graines, de divers végétaux et notamment de camomille, matricaire, épine-vinette, souci, centaurée, safran, artichaut, genêt, millepertuis, renouée, fougères, gaude, sumac, groseillier, verge d'or, ajonc, molène, carotte, séneçon, indigo, pastel, scabieuse, sophora, eucalyptus, myrtille, garance, orcanette, aspérule, carthame, vipérine, bourdaine, troène, petite centaurée, bouleau, frêne, genévrier, chêne, érable, châtaignier, théier, pin, nerprun, arbousier, lichens, campêche et les genres ou espèces voisines vendues sous cette appellation, pernambouc, gambier, henné, tournesol, curcuma, scutellaire, santal, aulne, rocouyer, cachou, safran, sanguinaire, tagète, gaillet, bois de Brésil, noyer, acacia, quinquina, cassia.

On utilise selon l'invention, et en fonction de la teinture recherchée, de préférence les extraits colorants liquides de fleurs de matricaire, feuilles de henné, bois de campêche ou genres voisins, bois de Pernambouc, bois de santal, fleurs de sophora, feuilles d'eucalyptus, feuilles d'indigo, racines de garance, fleurs de tagète, racines d'orcanette, lichens et champignons.

Les colorants naturels d'origine végétale qui peuvent être utilisés en association ou non aux extraits colorants d'origine végétale sont en particulier ceux présents dans les feuilles de henné, le bois de Pernambouc, le bois de campêche, les fruits de kamala, la racine de garance, les bogues de noyer, les fleurs de sophora, les feuilles
d'indigotier ou de pastel, les racines d'orcanette, le bois
de santal.

Parmi les colorants pouvant être utilisés dans les
compositions suivant l'invention, on peut citer par exemple : la maclurine (2,3', 4,4', 6 pentahydroxy benzophénone : C.I. Natural Yellow 11), la braziline (7,11 b-dihydrobenz [1,2-d] indéno [1,2-d] pyran 3, 6a, 9,10 (6H) -
tétrol ou C.I. Natural Red 24), l'hématoxyline (7,11 b-
dihydrobenz (b) - indéno [1,2-d] pyran 3,4 6a, 9, 10 (6H)
pentol) et ses dérivés (ou C.I. Natural Black 1 et 2),
l'alizarine (1,2 dihydroxy anthraquinone) et ses dérivés (ou
C.I. Natural Red 6,8, 9, 10, 11, 12), la purpuroxanthine et
purpurine (1, 2 , 4 trihydroxy anthraquinone ou C.I.
Natural Red 8 et 16), la juglone (5 hydroxy 1, 4 naphtoquinone ou C.I. Natural Brown 7), la lawsone (2 hydroxy 1, 4
naphtoquinone ou C.I. Natural Orange 6), l'acide kermésique
(acide 1, 2, 4, 7 tétrahydroxy 5 méthyl 6, anthraquinone
carboxylique ou C.I. Natural Red 3), la quercétine (3, 3',
4',5, 7 pentahydroxyflavone ou C.I. Natural Yellow 10), la
rutine (3,3', 4', 5, 7 pentahydroxyflavone 3 rutinoside ou
C.I. Natural Yellow 10), l'indigotine [$\Delta^{2, 2'}$ bilindoline]
3,3' dione ou C.I. Natural Blue 1), les catéchines (3,3',
4', 5, 7 flavonpentol ou C.I. Natural Brown 3), l'apigénine
(4', 5, 7 trihydroxyflavone ou C.I. Natural Yellow 1, 2),
les santalines ( ou C.I. Natural Red 22), la rotlérine (5, 7
dihydroxy - 2,2 diméthyl 6 [2, 4, 6 trihydroxy 3 méthyl 5
acétylbenzyl 8 - cynnamoyl] 1, 2 chromène ou C.I. Natural
Orange 2, Natural Yellow 25).

Selon l'invention, il est tout à fait possible
d'utiliser comme colorant un extrait colorant végétal activé
par son (ses) prinicpe(s) actif(s) colorant(s). Ainsi
l'extrait colorant de bois de campêche peut-il être activé
par son principe·actif, l'hématoxyline.

Selon l'invention, le(s) colorant(s) naturel(s) complexé(s) d'origine végétale a (ont) une concentration pouvant varier entre 0,01 et 30 % et de préférence entre 0,01 et 10 % en poids par rapport au poids total de la composition.

Les substances tinctoriales des extraits d'origine végétale tels que définis précédemment sont sous forme de complexes avec un ou plusiers cations métalliques, choisis parmi les cations naturels suivants : fer, aluminium, cobalt, zinc, cuivre, nickel, étain, etc...

Il est bien entendu que ces complexes, selon la présente invention, peuvent se présenter sous la forme de laques vraies.

Les cations métalliques, présents dans la composition, ont une teneur comprise entre 0,01 et 1 %, de préférence entre 0,02 et 0,6 %, en poids par rapport au poids total de la composition, teneur évitant des excès de sels métalliques.

Lorsqu'elles sont sous forme de complexes métalliques, les molécules colorantes ont des couleurs variables selon le type de cation complexant, cette propriété étant mise à profit pour faire varier les couleurs du cheveu ou du poil.

Selon la présente invention, les complexes métalliques cations - colorants sont stables et compatibles entre eux, procurant ainsi de très larges possibilités de mélanges entre les différents extraits colorants naturels d'origine végétale. Il est tout à fait possible par exemple d'utiliser un mélange composé d'un extrait de bois de campêche dont les principes actifs colorants sont complexés par le cation cobalt et d'un extrait de feuille de henné dont les principes colorants sont complexés par le cation aluminium.

Parmi les agents de pénétration présents selon l'invention dans les extraits colorants naturels d'origine végétale, nous pouvons citer les alcools et glycols de bas

poids moléculaire ($<C_{10}$) et leurs dérivés comme : l'éthanol, le méthanol, le butanol, le propanol, l'isopropanol, l'éthylène glycol, le butylène glycol, le propylène glycol, le dipropylène glycol, les carbitols, les cellosolves.

Les compositions, selon l'invention, sont caractérisées par le fait qu'elles sont toujours accompagnées d'un support dont le rôle est à la fois de faciliter l'application des extraits ou des colorants liquides d'origine végétale sur le cheveu ou le poil et d'optimiser leur pouvoir colorant afin d'obtenir une même reproductibilité des couleurs rendues.

Les supports peuvent être de trois types selon l'invention, mais de préférence un des deux premiers :

1) Un gel naturel aqueux constitué de substances végétales gélifiantes de type poly saccharidique : polyglucoses, xanthanes, mannanes, galactanes, carraghénates, gommes végétales, alginates, pectines. Le ou les extraits colorants d'origine végétale sont introduits dans le gel au moment de l'emploi, puis appliqués sur le cheveu ou le poil mouillé.

2) Une poudre insoluble, inerte, constituée de microsphères poreuses de silice enrobant le ou les extraits colorants d'origine végétale. Le produit obtenu possède alors l'aspect d'une poudre et peut être appliqué directement sur le cheveu ou le poil mouillé.

3) Tout produit cosmétique habituellement mis en contact avec les cheveux : shampooing, crème capillaire, beaume, lotions, gels, cataplasme, à condition que ce support présente les mêmes normes de pH que les deux autres supports précédemment cités.

Selon l'invention, l'application sur le cheveu ou le poil de la composition obtenue après mélange des extraits colorants et de leur support, est aisée pour toute personne, ne coule pas, et procure à la chevelure ou au poil une douceur et une brillance très satisfaisantes.

En plus des deux ingrédients principaux des compositions selon l'invention, celles-ci peuvent également contenir des adjuvants conventionnels tels que :

- des agents de conditionnement du cheveu ou du poil,
- des agents alcalinisants ou acidifiants, comme par exemple des acides organiques naturels, des carbonates alcalins,
- des agents épaississants naturels,
- des agents mouillants,
- des agents conservateurs,
- des essences naturelles ou des parfums.

Le ou les agents liquides de pénétration sont présents dans la composition à une teneur comprise entre 0,1 et 20 %, de préférence entre 1 et 10 %, en poids par rapport au poids total de la composition.

Selon l'invention, leur rôle comprend à la fois la fonction d'agent de pénétration, d'agent solvant et d'agent conservateur du produit.

La présente invention a également pour objet un procédé de teinture des cheveux ou du système pileux en général, consistant à appliquer sans dilution, sur une partie ou l'ensemble de la chevelure ou des poils, une composition telle que décrite ci-dessus, à laisser agir à froid la dite composition pendant un temps compris entre 3 minutes et 2 heures, puis à rincer les cheveux ou les poils à l'eau courante ou à l'aide de tout produit de rinçage préconisé à cette fin.

Le rinçage peut être éventuellement suivi d'un shampooing ou d'un beaume démêlant ou conditionneur afin d'obtenir un meilleur résultat.

A titre d'illustration et sans aucun caractère limitatif, nous décrivons plusieurs exemples de compositions selon l'invention :

Exemple 1

a) - Extrait de bois de campêche liquide titré en hématoxyline/hématéine sous forme de complexe avec $Co^{++}$ ............. 6,5 ml

- n Butanol ....................... 1,5 ml

- Cellosolve ...................... 2,0 ml

                                                                10   ml

b) - Conservateur ...................... 0,1 ml

- Essence végétale naturelle ........ 0,05 ml

- Gel aqueux à 2 % de polyglucose q.s.p. 100   ml

Le mélange homogène obtenu après l'addition de a dans b est brun rouge, de pH 4,0. Cette composition, appliquée pendant 30 minutes à froid, sur une chevelure naturelle blanche ou blonde ou châtain, colore les cheveux en noir après rinçage à l'aide d'une solution de carbonate de sodium à 2,5 % dans l'eau. La couleur obtenue est un noir naturel très esthétique, résistant pendant une période de six semaines environ. La coloration ainsi obtenue peut être accentuée par une seconde application ou diminuée par des lavages à l'aide de shampooings classiques. L'élimination de la teinture permet de retrouver la couleur naturelle du cheveu sans effet désagréable (changement de teinte) ni altération du cheveu.

Exemple 2

a) Extrait de camomille matricaire liquide , titré en flavonoïdes sous forme de complexes avec le cation aluminium ......................... 6,0 ml

- Quercétine en solution à 1,25 % ..... 1,0 ml

- n Butanol ......................... 1,0 ml

- Cellosolve ........................ 0,5 ml

- Propylène glycol .................. 1,5 ml

b) - Essence végétale .................... 0,1 ml

- Conservateur ....................... 0,1 ml

- Agent conditionneur du cheveu :
dérivés de graines de guarée ........ 1,0 ml

- Gel aqueux à 2 % de polyglucose
naturel .................... q.s.p. 100 ml

Avant son application sur la chevelure, l'extrait a est mélangé à son support b de façon homogène. La composition obtenue, de couleur jaune franc et à un pH de 5,0, est appliquée sur les cheveux ou les poils pendant un temps compris entre 30 et 40 minutes puis rincée à l'eau courante.

Sur des cheveux châtains foncés ou noirs, un éclaircissement très prononcé de la teinte est obtenu, correspondant à une nuance.

Sur des cheveux blonds ou châtains clairs, une belle couleur blond doré très lumineuse est observée. Plusieurs applications de la composition conduisent à un effet plus accentué ; de même, l'utilisation de plusieurs lavages à l'aide de shampooings permettent de retrouver la couleur initiale du cheveu ou du poil.

Exemple 3

a) - Extrait de bois de campêche liquide
titré en hématoxyline/hématéine sous
forme de complexes avec le cation
cobalt                                          4,5 ml

- Extrait de sophora japonica liquide,
titré en flavonoïdes sous forme de
complexes avec le cation aluminium ... 2,0 ml

- Hématoxyline à 2,5 % ................. 0,5 ml

- Quercétine à 1,25 % ................. 1,0 ml

- n Butanol .......................... 1,0 ml

- Cellosolve ......................... 1,0 ml

- Essence végétale ................... 0,1 ml

b) - Poudre de silice microporeuse en
sphérules de 5 microns ................ 70 g

Cette composition obtenue après mélange de a et b est une "poudre" enrobant l'extrait végéral colorant. Le mélange obtenu, appliqué sur le cheveu ou le poil mouillé, relargue l'extrait végétal colorant au contact de l'eau. Après 30 et 40 minutes d'application sur une chevelure ou des poils blonds ou châtains très clairs et rinçage à l'eau courante, on obtient une magnifique teinture châtain clair. Les cheveux ont du corps, ne sont absolument pas abîmés et conservent leur teinte pendant 5 à 6 semaines.

Exemple 4

a) - Extrait de bois de campêche liquide
titré en hématoxyline/hématéine sous
forme de complexes avec le cation
cobalt ........................... 4,0 ml
- Extrait de bois de santal liquide titré
en santalines, sous forme de complexes
avec le cation aluminium ........... 2,0 ml
- Extrait de feuilles d'indigo titré en
indigotine sous forme de complexes
avec l'aluminium .................... 1,0 ml
- Hématoxyline à 2,5 % .............. 0,2 ml
- Indigotine à 2,5 % ............... 0,3 ml
- Rutine à 2 % ..................... 1,0 ml
- n Butanol ........................ 0,5 ml
- Cellosolve ....................... 1,0 ml

b) - Shampooing composé de :
- Extrait de bois de Panama .......... 70 ml
- Extrait de saponaire .............. 28,7ml
- Acide citrique à 40 % ............. 0,1ml
- Parfum ........................... 0,2ml

- Agent conditionneur :

    dérivés de graine de guarée ......... 1 ml

Avant son application sur le cheveu ou le poil, on mélange l'extrait colorant à raison de 100 ml de shampooing. La composition ainsi obtenue de pH 6,0 et de consistance liquide est appliquée sur le cheveu ou le poil durant une période de 25 minutes.

Après rinçage à l'eau courante et séchage, une chevelure blonde ou châtain clair prend une teinte châtain foncé de très belle allure. La teinture obtenue par cette composition s'efface après 5 ou 6 shampooings sans modification de la couleur naturelle du cheveu ou du poil.

Exemple 5

    a) - Extrait d'eucalyptus macroryncha
        titré en quercétine sous forme de
        complexes avec le cation aluminium .. 3,0 ml
        - Juglone à 0,5 % ..................... 0,5 ml
        - Quercétine à 1,25 % ................. 1,0 ml
        - Extrait de feuilles de noyer ........ 1,5 ml
        - Extrait de feuilles de thé .......... 1,5 ml
        - Catéchine à 1,5 % ................... 0,5 ml
        - Alcool éthylique .................... 1,5 ml
        - Carbitol ............................ 0,5 ml
    b) - Poudre de silice microporeuse en
        sphérules de 7 microns .......... 70 grammes

La composition obtenue après mélange de a et b est une "poudre" enrobant l'extrait végétal colorant. Le mélange ainsi obtenu est appliqué pendant une période de 40 minutes sur cheveux blonds.

Après rinçage à l'eau courante et séchage, les cheveux ou les poils prennent une magnifique teinte blond cendré.

L'indice d'irritation oculaire chez le lapin, déterminé selon le test de Draize (méthode officielle française, arrêtés des 5 avril 1971 et 16 avril 1973) ainsi que

0124393

l'indice d'irritation primaire cutanée déterminé chez le lapin selon la méthode officielle française (arrêté du 1er février 1982) montrent que les extraits colorants végétaux utilisés selon l'invention sont non irritants, même à l'état pur.

REVENDICATIONS

1. Compositions cosmétiques sous forme d'extraits colorants naturels et liquides destinées à la teinture directe semi-permanente et réversible des cheveux et du système pileux, réalisable à froid, caractérisées par le fait qu'elles sont constituées de :

a) au moins un extrait colorant et/ou des colorants d'origine végétale dont les molécules tinctoriales sont sous forme de complexes avec un ou plusieurs cations métalliques rigoureusement dosés pour éviter tout excès de cation métallique en liberté, associé à un ou plusieurs agents de pénétration liquides,

b) au moins un support d'origine naturelle permettant une application efficace du complexe colorant.

2. Composition suivant la revendication 1, caractérisée par le fait qu'elle contient un ou plusieurs extraits végétaux colorants dans une proportion comprise entre 0,01 et 50 %, de préférence entre 1 et 20 %, en poids par rapport au poids total de la composition.

3. Composition suivant l'une des revendications 1 et 2, caractérisée par le fait que l'extrait colorant d'origine végétale est obtenu par extraction du bois de campêche et de toutes les espèces végétales vendues sous cette appellation , de feuilles de henné, de fleurs de matricaire, de fleurs de sophora, de feuilles d'eucalyptus, de racines de garance, de racines d'orcanette, de bois de Pernambouc, de feuilles d'indigo ou de pastel, de bois de santal, de fleurs de tagète, de lichens, de champignons ou de toute plante tinctoriale répertoriée.

4. Composition suivant l'une quelconque des revendications précédentes, caractérisée par le fait qu'elle contient en outre un ou plusieurs colorants purifiés d'origine naturelle, pris dans le groupe constitué par la maclurine, la braziline, la braziléine, l'hématoxyline, l'héma- téine, l'alizarine, la purpuroxanthine et ses dérivés, la juglone, la lawsone, les catéchines, épicatéchines et leurs polymères, les acides gallique, ellagique et leurs polymères, les flavonoïdes du groupe des flavones, flavonols, flavanones, isoflavones et flavononols, les santalines, la rottlérine, la rubiadine, la purpurine et ses dérivés, l'émodine, le lapachol, les caroténoïdes et xanthophylles, l'alkanine, la butéine et la butine, l'indigotine, l'indirubine, le pratol, la chiconine, la bixine.

5. Composition suivant l'une quelconque des revendications précédentes, caractérisée par le fait que les substances tinctoriales de l'extrait d'origine végétale sont complexées par un ou plusieurs cations métalliques d'origine naturelle, rigoureusement dosés, choisis parmi les cations suivants :
fer, aluminium, cobalt, zinc, cuivre, étain, nickel, pouvant conduire ou non à la formation de laques.

6. Composition suivant la revendication 5, caractérisée par le fait que les colorants végétaux complexés par les cations métalliques sont présents dans la composition à une teneur comprise entre 0,01 et 30 %, et de préférence entre 0,01 et 10 %, en poids par rapport au poids total de la composition.

7. Composition suivant l'une des revendications 5 et 6, caractérisée par le fait que le ou les cations métalliques sont présents dans la composition à une teneur comprise entre 0,01 et 1 % et, de préférence, entre 0,02 et 0,6 % en poids par rapport au poids total de la composition, teneur évitant tout excès de sels métalliques.

8. Composition suivant l'une quelconque des revendications précédentes, caractérisée par le fait que le ou les agents de pénétration sont choisis parmi les alcools ou glycols de bas poids moléculaires ($<C_{10}$), du type éthanol, méthanol, butanol, propanol, isopropanol, etc..., éthylène glycol, propylène glycol, butylène glycol, dipropylène glycol et leurs dérivés, le ou les agents liquides de pénétration présents dans la composition ayant une teneur comprise entre 0,1 et 20 % et, de préférence, entre 1 et 10 % en poids par rapport au poids total de la composition.

9. Composition suivant l'une quelconque des revendications précédentes, caractérisée par le fait que le support d'application sur le cheveu ou le système pileux peut être ou bien une poudre insoluble inerte constituée de microsphères poreuses de silice enrobant l'extrait colorant végétal liquide, ou bien un gel naturel constitué de substances végétales gélifiantes du type polysaccharides en solution aqueuse, ou bien tout produit cosmétique habituellement mis en contact avec les cheveux ou le système pileux et notamment shampooing, crème capillaire, beaume, lotions, gels, cataplasmes.

10. Composition suivant l'une quelconque des revendications précédentes, caractérisée par le fait qu'elle contient un agent de conditionnement du cheveu et du système pileux en général, le dit agent étant choisi parmi les dérivés des protéines végétales ou animales, les ammoniums quaternaires et leurs dérivés, résines de condensation de polyglycols polyamines, etc...

11. Composition suivant l'une quelconque des revendications précédentes, caractérisée par le fait qu'elle contient des adjuvants conventionnels tels que des agents alcalinisants ou acidifiants, des agents épaississants, des agents traitants ou de conditionnement du cheveu ou du système pileux, des agents mouillants, des agents conservateurs, des essences naturelles ou des parfums.

12. Procédé pour la teinture des cheveux et du système pileux en général, caractérisé par le fait qu'il consiste à appliquer sans dilution directement sur le cheveu ou le poil humide, une composition telle que revendiquée selon l'une quelconque des revendications 1 à 11, à laisser agir à froid la dite composition pendant un temps pouvant être compris entre 3 minutes et 2 heures, puis à rincer les cheveux ou les poils à l'eau courante ou à l'aide de tout produit de rinçage préconisé à cet effet.

13. Procédé selon la revendication 12, caractérisé par le fait que la composition prête à l'emploi présente un pH compris entre 2,5 et 12 et, de préférence, entre 3 et 8.

14. Procédé selon l'une des revendications 12 et 13, caractérisé par le fait que, pour rendre la teinture obtenue parfaitement réversible, on effectue des lavages successifs à l'aide de shampooings ou bases détergentes classiques.

0124393

Office européen
des brevets

**RAPPORT DE RECHERCHE EUROPEENNE**

Numéro de la demande

EP 84 40 0609

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| | | | A 61 K 7/13 |
| X | US-A-4 190 064 (GORDON et al.)  * revendications; colonne 3, ligne 2 - colonne 7, ligne 44 * | 1-4,8-14 | |
| X | FR-A-2 500 748 (L'OREAL)  * revendications; page 3, ligne 15 - page 4, ligne 15; page 4, lignes 32-40; page 5, lignes 10-31; page 6, lignes 3-17,25 - page 7, ligne 12; exemples * | 1-4,8-14 | |
| X | EP-A-0 072 298 (P. FABRE)  * revendications; page 7, lignes 11-28; exemples * | 1-4,8-14 | |
| | | | **DOMAINES TECHNIQUES RECHERCHES (Int. Cl. ³)** |
| Y | FR-A-2 483 228 (P. FABRE)  * revendications; page 2, lignes 36-39; exemple C * | 1,3,5, 6,7,9 | A 61 K 7/00 |

Le présent rapport de recherche a été établi pour toutes les revendications

| Lieu de la recherche | Date d'achèvement de la recherche | Examinateur |
|---|---|---|
| LA HAYE | 20-07-1984 | WILLEKENS G.E.J. |